# EUROPEAN PATENT SPECIFICATION

(11) **EP 2 207 545 B1**
(45) Date of publication and mention of the grant of the patent: **06.01.2016**
(21) Application number: 09733327.2
(22) Date of filing: 17.04.2009
(51) Int. Cl.: A61K 31/415, A61P 25/08

(54) **CLONIDINE FOR USE IN THE TREATMENT OF MUSCLE SPASTICITY ASSOCIATED WITH SURGERY**
CLONIDIN ZUR BEHANDLUNG VON CHIRURGIE VERBUNDENEN SPASTIZITÄT
CLONIDINE DESTINÉE AU TRAITEMENT DE LA SPASTICITÉ ASSOCIÉE AVEC LA CHIRURGIE

(30) Priority: 18.04.2008 US 46192 P; 20.03.2009 US 408491
(43) Date of publication of application: 21.07.2010
(73) Proprietor: Warsaw Orthopedic, Inc., Warsaw, IN 46581 (US)
(72) Inventor: KING, Vanja, Margareta, Memphis TN 38104 (US)
(74) Representative: O'Connell, Maura
(86) International application number: PCT/US2009/040885
(87) International publication number: WO 2009/129428

(56) References cited:
- US-A1- 2003 229 088
- US-A1- 2005 059 744
- US-A1- 2007 196 458
- US-A1- 2007 243 228
- US-A1- 2008 019 975
- PETER KRANKE ET AL: "Single-Dose Parenteral Pharmacological Interventions for the Prevention of Postoperative Shivering: A Quantitative Systematic Review of Randomized Controlled Trials", ANESTHESIA & ANALGESIA, vol. 99, no. 3, 1 September 2004 (2004-09-01), pages 718-727, XP55016183, ISSN: 0003-2999, DOI: 10.1213/01.ANE.0000130589.00098.CD
- CLAUDIA STAPELFELDT ET AL: "Intraoperative Clonidine Administration to Neurosurgical Patients", ANESTHESIA & ANALGESIA, vol. 100, no. 1, 1 January 2005 (2005-01-01), pages 226-232, XP55016184, ISSN: 0003-2999, DOI: 10.1213/01.ANE.0000142122.57201.6B
- I. VANDERSTAPPEN ET AL: "The effect of prophylactic clonidine on postoperative shivering.", ANAESTHESIA, vol. 51, no. 4, 1 April 1996 (1996-04-01), pages 351-355, XP55016186, ISSN: 0003-2409, DOI: 10.1111/j.1365-2044.1996.tb07747.x

## Description

### BACKGROUND

Shivering is a bodily function in response to early hypothermia in warm-blooded animals. When the core body temperature drops, the shivering reflex is triggered. Muscle groups around the vital organs begin to shake in small movements in an attempt to create warmth by expending energy. Shivering can be associated with surgery or result after surgery in which case it is known as post-surgical shivering.

Spasticity is a severe discomfort that is distinct from incisional or neuropathic pain It is a disorder of the central nervous system in which certain muscles are continuously contracted. This contraction causes stiffness or tightness of the muscles and may interfere with movement, speech, *etc.*; it can be very painful and frightening for the patient. Moreover, after surgery, muscle spasticity can delay post-operative recovery and often can extend the hospital stay of the patient.

Muscle spasticity may result from a number of causes. For example, it may be a recurring state that accompanies conditions such as cerebral palsy, which is due to brain damage. It also may be caused by environmental strains or trauma such as the stress imposed on the body that follows or is incidental to surgery. This type of strain or trauma differs from other types of spasticity both because it is not caused by brain damage and because to a degree it is predictable with respect to whom it most likely will affect, is most likely to occur within a finite window of time, and is localized to a site at or near the site of a surgical procedure. Known pharmaceutical treatments for muscle spasticity from brain damage include baclofen, diazepam, dantrolene and clonazepam.

Another pharmaceutical that is known to the medical profession for treating spasticity due to brain damage is clonidine, which is widely recognized as an antihypertensive agent that acts as an agonist on the alpha-2-adrenergic receptor and a neural receptor agonist. In general, clonidine, also referred to as 2,6-dichloro-N-2-imidazolidinyldenebenzenamine (C₉H₉Cl₂N₃) may be represented by the following chemical structure:

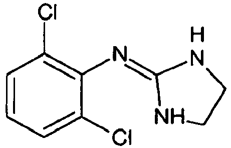

However, to date it has not been appreciated as an effective treatment for spasticity that accompanies post-operative surgery and which is not due to brain damage but direct injury to muscles and nerves. This injury to the muscles includes both direct incision of the muscle and the separation of muscle fibers that occur in minimally invasive surgeries, but which does not include cutting of the muscle fibers.

Because of the unique manifestation and relatively predictable risks for post-operative spasticity, there is a need for effective treatments for this condition, including methods and compositions to alleviate or to treat post-operative spasticity. Another side effect of surgery is post-operative shivering. Clonidine has demonstrated efficacy in reducing or eliminating post-operative shivering. A depot formulation for treatment of muscle spasticity would also provide control of post-operative shivering.

### SUMMARY

Clonidine or its pharmaceutically acceptable salt according to the invention are administered in order to relax muscles after surgery. As noted above, clonidine is recognized as an alpha-2-adrenergic receptor agonist, and a neural receptor agonist, and although it is most well known as a treatment for hypertension, an advantage of the present invention is that it takes this known compound and when administered in an effective amounts formulation, particularly in sustain release formulations, it may provide an effective treatment for spasticity that is incidental to surgery.

According to one embodiment, there is provided a clonidine or a pharmaceutically acceptable salt thereof for use in treating a mammal suffering from muscle spasticity associated with surgery, said use comprising administering a therapeutically effective amount of clonidine or a pharmaceutically acceptable salt thereof and a biodegradable polymer selected from D,L-lactide or L-lactide-caprolactone at a target tissue site beneath the skin to relax muscle at or near the target tissue site wherein clonidine is for administration locally to the target tissue site as a drug depot with a layer thickness of from 0.005mm to 1.0mm in the form of a ribbon-like fiber that releases the clonidine over a period of time.

Ideally, the clonidine or a pharmaceutically acceptable salt thereof is capable of being administered to a post-operative surgery site.

According to another embodiment, the clonidine is provided in a sustain release composition comprising an effective amount of clonidine or a pharmaceutically acceptable salt thereof in an implantable drug depot, wherein said clonidine is present in an amount to relieve spasticity for a period of 5-12 days or 7-10 days and said implantable drug depot is present in an amount that facilitates sustain release of clonidine over said period.

According to another embodiment, the clonidine is provided in an immediate release composition comprising an effective amount of clonidine or a pharmaceutically acceptable salt thereof in a drug depot, wherein said clonidine is present in an amount to relieve spasticity and said drug depot is present in an amount that initially facilitates immediate release of clonidine.

According to another embodiment, the clonidine is for use in treating a mammal suffering from muscle spasticity associated with surgery, said method comprising administering a therapeutically effective amount of clonidine at a target site beneath the skin to relax muscle at or near the target site, wherein said therapeutically effective amount prevents the onset of spasticity.

The surgery may for example be minimally invasive or open. Further, in some embodiments, the clonidine is or is administered as part of a therapeutic agent that is a sustain release formulation that can be released over 5 - 12 days or 7 - 10 days and thereby be present during the periods in which spasticity is likely to occur. In some embodiments the release is approximately 8% to 20% per day, and in some embodiments, the release is approximately 5%-15% per day.

According to another embodiment, clonidine or a pharmaceutically acceptable salt thereof is used for treating a mammal for muscle spasticity, wherein said mammal has undergone surgery at a target sitein the form of a dosage amount and with a release rate profile sufficient to relax muscle fibers at or near the target site.

The clonidine is administered as part of a drug depotas a ribbon-like fiber.

The clonidine or pharmaceutically acceptable salt thereof also may be co-administered with an anti-inflammatory agent, an analgesic agent, a skeletal muscle relaxant, an osteoinductive anabolic growth factor, an anti-catabolic growth factor or a combination thereof.

In some embodiments, the clonidine or pharmaceutically acceptable salt thereof is administered in a sustain release formulation alone, and in other embodiments it is administered in part as a bolus suspended for example in a gel to provide an immediate release of the therapeutic agent and in part as a sustain release formulation. The effective amount of the therapeutic agent that is part of the sustain release formulation may for example be encapsulated in a plurality of microparticles, microspheres, microcapsules and/or microfibers to provide sustained release of the therapeutic agent over time. The sustain release and immediate release formulations may be used in combination and introduced to the body at the same time, with the immediate release formulation be accessible first for the treatment of spasticity.

In yet another exemplary embodiment, the clonidine or a pharmaceutically acceptable salt thereof is delivered into a target tissue site beneath the skin. The method comprises inserting a cannula at or near a target tissue site and injecting a gel capable of adhering to the target tissue site, the gel comprising one or more biodegradable depots containing an effective amount of clonidine, wherein the target tissue site comprises a spinal disc, spinal foraminal space near the spinal nerve root, facet, spinal canal, or bone.

In another embodiment, an implantable drug depot is provided. The drug depot may comprise: (i) one or more immediate release layers that release a bolus dose of clonidine or a pharmaceutically acceptable salt thereof at a site beneath the skin; and (ii) one or more sustain release layers that release an effective amount of clonidine or a pharmaceutically acceptable salt thereof over a period of 5 to 12 days.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description and appended claims.

### BRIEF DESCRIPTION OF THE DRAWINGS

In part, other aspects, features, benefits and advantages of the embodiments will be apparent with regard to the following description, appended claims and accompanying drawings where:
Figure 1 illustrates a number of common locations within a patient that may be sites at which surgery takes place and locations at which the drug depot containing clonidine can locally be administered thereto and used to treat spasticity.
Figure 2 illustrates a schematic dorsal view of the spine and sites where the drug depot containing clonidine can locally be administered thereto.

It is to be understood that the figures are not drawn to scale. Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

### DETAILED DESCRIPTION

Notwithstanding that the numerical ranges and parameters setting forth, the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, *e.g.,* 5.5 to 10.

It is noted that, as used in this specification and the appended claims, the singular forms "a," "an," and "the," include plural referents unless expressly and unequivocally limited to one referent. Thus, for example, reference to "a drug depot" includes one, two, three or more drug depots.

Reference will now be made in detail to certain embodiments of the invention, examples of which are illustrated in the accompanying drawings. While the invention will be described in conjunction with the illustrated embodiments, it will be understood that they are not intended to limit the invention to those embodiments. On the contrary, the invention is intended to cover all alternatives, modifications, and equivalents that may be included within the invention as defined by the appended claims.

The headings below are not meant to limit the disclosure in any way; embodiments under any one heading may be used in conjunction with embodiments under any other heading.

Unless otherwise specified or apparent from context, where this specification and the set of claims that follows refer to clonidine, the inventors are also referring to a pharmaceutically acceptable salts of clonidine. In various embodiments, the clonidine includes pharmaceutically acceptable salts. Some examples of pharmaceutically acceptable salts include those salt-forming acids and bases that do not substantially increase the toxicity of the compound. Some examples of suitable salts include salts of alkali metals such as magnesium, potassium and ammonium. Salts of mineral acids such as hydrochloric, hydriodic, hydrobromic, phosphoric, metaphosphoric, nitric and sulfuric acids, as well as salts of organic acids such as tartaric, acetic, citric, malic, benzoic, glycollic, gluconic, gulonic, succinic, arylsulfonic, *e.g.,* p-toluenesulfonic acids. Exemplary formulations may use either the base or the hydrochloric salt of clonidine.

The compositions of described herein are not limited to uses in connection with any specific surgery and include spasticity that may be associated with arthroscopic surgery, laparoscopic surgery, open back surgery and oral surgery.

### Drug Depot

A drug depot is the composition in which the clonidine is administered to the body. Thus, a drug depot may comprise a physical structure to facilitate implantation and retention in a desired site *(e.g.,* a disc space, a spinal canal, a tissue of the patient, particularly at or near a site of surgery, *etc.).* The drug depot also comprises the drug itself. The term "drug" as used herein is generally meant to refer to any substance that alters the physiology of a patient. The term "drug" may be used interchangeably herein with the terms "therapeutic agent", "therapeutically effective amount", and "active pharmaceutical ingredient" or "API." It will be understood that unless otherwise specified a "drug" formulation may include more than one therapeutic agent, wherein exemplary combinations of therapeutic agents include a combination of two or more drugs. The drug provides a concentration gradient of the therapeutic agent for delivery to the site. In various embodiments, the drug depot provides an optimal drug concentration gradient of the therapeutic agent at a distance of up to about 1 cm to about 5 cm from the implant site, and comprises clonidine.

A "therapeutically effective amount" or "effective amount" is such that when administered, the drug results in alteration of the biological activity, such as, for example, inhibition of inflammation, reduction or alleviation of pain or spasticity, improvement in the condition through muscle relaxation, *etc.* The dosage administered to a patient can be as single or multiple doses depending upon a variety of factors, including the drug's administered pharmacokinetic properties, the route of administration, patient conditions and characteristics (sex, age, body weight, health, size, *etc.),* extent of symptoms, concurrent treatments, frequency of treatment and the effect desired. In some embodiments the formulation is designed for immediate release. In other embodiments the formulation is designed for sustained release. In other embodiments, the formulation comprises one or more immediate release surfaces and one or more sustained release surfaces, or by using a mixture of formulations which provide different release profiles, either by use of different forms of the drug or by mixtures of different formulations of sustained release materials. As persons of ordinary skill are aware a sustain release (or "sustained release") formulation is a formulation that permits the active ingredient to become accessible over a period of time, e.g. hours or days at a desired rate. By contrast, an immediate release formulation is accessible immediately or essentially immediately. The two types of formulation may be used in conjunction. For example a bolus or immediate release formulation of clonidine may be placed at or near the surgery site and a sustain release formulation may also be placed at or near the same site, or be provided within the same formulation through a combination of different polymer matrices and/or drug forms. Thus, even after the bolus becomes completely accessible, the sustained release formulation would continue to provide the active ingredient for the intended tissue.

The clonidine or its pharmaceutically acceptable salt may be administered with another muscle relaxant. Exemplary muscle relaxants include by way of example alcuronium chloride, atracurium bescylate, baclofen, carbolonium, carisoprodol, chlorphenesin carbamate, chlorzoxazone, cyclobenzaprine, dantrolene, decamethonium bromide, fazadinium, gallamine triethiodide, hexafluorenium, meladrazine, mephensin, metaxalone, methocarbamol, metocurine iodide, pancuronium, pridinol mesylate, styramate, suxamethonium, suxethonium, thiocolchicoside, tizanidine, tolperisone, tubocuarine, vecuronium, or combinations thereof.

The drug depot may comprise therapeutic agents in addition to the clonidine. Therapeutic agents, in various embodiments, block the transcription of translation of TNF-a or other proteins in the inflammation cascade. Suitable therapeutic agents include, but are not limited to, integrin antagonists, alpha-4 beta-7 integrin antagonists, cell adhesion inhibitors, interferon gamma antagonists, CTLA4-Ig agonists/antagonists (BMS-188667), CD40 ligand antagonists, Humanized anti-IL-6 mAb (MRA, Tocilizumab, Chugai), HMGB-1 mAb (Critical Therapeutics Inc.), anti-IL2R antibodies (daclizumab, basilicimab), ABX (anti IL-8 antibodies), recombinant human IL-10, or HuMax IL-15 (anti-IL 15 antibodies).

Other suitable therapeutic agents include IL-1 inhibitors, such Kineret® (anakinra) which is a recombinant, non-glycosylated form of the human inerleukin-1 receptor antagonist (IL-1Ra), or AMG 108, which is a monoclonal antibody that blocks the action of IL-1. Therapeutic agents also include excitatory amino acids such as glutamate and aspartate, antagonists or inhibitors of glutamate binding to NMDA receptors, AMPA receptors, and/or kainate receptors. Interleukin-1 receptor antagonists, thalidomide (a TNF-α release inhibitor), thalidomide analogues (which reduce TNF-α production by macrophages), bone morphogenetic protein (BMP) type 2 and BMP-4 (inhibitors of caspase 8, a TNF-α activator), quinapril (an inhibitor of angiotensin II, which upregulates TNF-α), interferons such as IL-11 (which modulate TNF-α receptor expression), and aurin-tricarboxylic acid (which inhibits TNF-α), for example, may also be useful as therapeutic agents for reducing inflammation. It is contemplated that where desirable a pegylated form of the above may be used. Examples of other therapeutic agents include NF kappa B inhibitors such as glucocorticoids, antioxidants, such as dilhiocarbamate, and other compounds, such as, for example, sulfasalazine.

Specific examples of therapeutic agents suitable for use include an anti-inflammatory agent, analgesic agent, or osteoinductive growth factor or a combination thereof. Anti-inflammatory agents include salicylates, diflunisal, sulfasalazine, indomethacin, ibuprofen, naproxen, tolmetin, ketorolac, diclofenac, ketoprofen, fenamates (mefenamic acid, meclofenamic acid), enolic acids (piroxicam, meloxicam), nabumetone, celecoxib, etodolac, nimesulide, apazone, gold, sulindac or tepoxalin; antioxidants, such as dithiocarbamate, and other compounds such as sulfasalazine [2-hydroxy-5-[-4-[C2-pyridinylamino)sulfonyl]azo]benzoic acid], steroids, such as fluocinolone, cortisol, cortisone, hydrocortisone, fludrocortisone, prednisone, prednisolone, methylprednisolone, triamcinolone, betamethasone, dexamethasone, beclomethasone, fluticasone or a combination thereof.

Suitable anabolic growth or anti-catabolic growth factors include a bone morphogenetic protein, a growth differentiation factor, a LIM mineralization protein, CDMP or progenitor cells or a combination thereof.

Suitable analgesic agents include acetaminophen, lidocaine, bupivicaine, opioid analgesics such as buprenorphine, butorphanol, dextromoramide, dezocine, dextropropoxyphene, diamorphine, fentanyl, alfentanil, sufentanil, hydrocodone, hydromorphone, ketobemidone, levomethadyl, mepiridine, methadone, morphine, nalbuphine, opium, oxycodone, papaveretum, pentazocine, pethidine, phenoperidine, piritramide, dextropropoxyphene, remifentanil, tilidine, tramadol, codeine, dihydrocodeine, meptazinol, dezocine, eptazocine, flupirtine or a combination thereof.

Analgesics also include agents with analgesic properties, such as for example, amitriptyline, carbamazepine, gabapentin, pregabalin, or a combination thereof.

The depot may contain a muscle relaxant. Exemplary muscle relaxants include by way of example alcuronium chloride, atracurium bescylate, baclofen, carbolonium, carisoprodol, chlorphenesin carbamate, chlorzoxazone, cyclobenzaprine, dantrolene, decamethonium bromide, fazadinium, gallamine triethiodide, hexafluorenium, meladrazine, mephensin, metaxalone, methocarbamol, metocurine iodide, pancuronium, pridinol mesylate, styramate, suxamethonium, suxethonium, thiocolchicoside, tizanidine, tolperisone, tubocuarine, vecuronium, or combinations thereof.

The clonidine may also be administered with non-active ingredients. These non-active ingredients may have multi-functional purposes including the carrying, stabilizing and controlling the release of the therapeutic agent(s). The sustained release process, for example, may be by a solution-diffusion mechanism or it may be governed by an erosion-sustained process. Typically, the depot will be a solid or semi-solid formulation comprised of a biocompatible material, which can be biodegradable. The term "solid" is intended to mean a rigid material, while, "semi-solid" is intended to mean a material that has some degree of flexibility, thereby allowing the depot to bend and conform to the surrounding tissue requirements.

In various embodiments, the non-active ingredients will be durable within the tissue site for a period of time equal to (for biodegradable components) or greater than (for non-biodegradable components) the planned period of drug delivery. For example, the depot material may have a melting point or glass transition temperature close to or higher than body temperature, but lower than the decomposition or degradation temperature of the therapeutic agent. However, the pre-determined erosion of the depot material can also be used to provide for slow release of the loaded therapeutic agent(s).

In various embodiments, the clonidine may have a high drug loading, such that the therapeutic agent comprises about 5-99 wt % of a depot, or 30-95 wt % of a depot, or 50-95 wt % of a depot. The balance is depot material, including optional inactive materials.

In some instance, it may be desirable to avoid having to remove the drug depot after use. In those instances, the depot may comprise a biodegradable material. There are numerous materials available for this purpose and having the characteristic of being able to breakdown or disintegrate over a prolonged period of time when positioned at or near the target tissue. As a function of the chemistry of the biodegradable material, the mechanism of the degradation process can be hydrolytical or enzymatical in nature, or both. In various embodiments, the degradation can occur either at the surface (heterogeneous or surface erosion) or uniformly throughout the drug delivery system depot (homogeneous or bulk erosion).

A "depot" includes but is not limited to capsules, (micro)spheres, (micro)particles, (micro)capsules, (micro)fibers, particles, nanospheres, nanoparticles, coating, matrices, wafers, pills, pellets, emulsions, liposomes, micelles, gels, or other pharmaceutical delivery compositions or a combination thereof. Suitable materials for the depot are ideally pharmaceutically acceptable biodegradable and/or any bioabsorbable materials that are preferably FDA approved or GRAS materials. These materials can be polymeric or non-polymeric, as well as synthetic or naturally occurring, or a combination thereof.

The term "biodegradable" includes that all or parts of the drug depot will degrade over time by the action of enzymes, by hydrolytic action and/or by other similar mechanisms in the human body. In various embodiments, "biodegradable" includes that the depot *(e.g.,* microparticle, microsphere, *etc.)* can break down or degrade within the body to non-toxic components after or while a therapeutic agent has been or is being released. By "bioerodible" it is meant that the depot will erode or degrade over time due, at least in part, to contact with substances found in the surrounding tissue, fluids or by cellular action. By "bioabsorbable" it is meant that the depot will be broken down and absorbed within the human body, for example, by a cell or tissue. "Biocompatible" means that the depot will not cause substantial tissue irritation or necrosis at the target tissue site.

The depot comprises a bioabsorbable, a bioabsorbable, and/or a biodegradable biopolymer that may provide immediate release, or sustained release of the clonidine selected from D,L-lactide, or L-lactide-caprolactoneor combinations thereof.

The depot may optionally contain inactive materials such as buffering agents and pH adjusting agents such as potassium bicarbonate, potassium carbonate, potassium hydroxide, sodium acetate, sodium borate, sodium bicarbonate, sodium carbonate, sodium hydroxide or sodium phosphate; degradation/release modifiers; drug release adjusting agents; emulsifiers; preservatives such as benzalkonium chloride, chlorobutanol, phenylmercuric acetate and phenylmercuric nitrate, sodium bisulfite, sodium bisulfate, sodium thiosulfate, thimerosal, methylparaben, polyvinyl alcohol and phenylethyl alcohol; solubility adjusting agents; stabilizers; and/or cohesion modifiers. Typically, any such inactive materials will be present within the range of 0-75 wt %, and more typically within the range of 0-30 wt %. If the depot is to be placed in the spinal area, in various embodiments, the depot may comprise sterile preservative free material.

The depot can be different sizes, shapes and configurations. There are several factors that can be taken into consideration in determining the size, shape and configuration of the drug depot. For example, both the size and shape may allow for ease in positioning the drug depot at the target tissue site that is selected as the implantation or injection site. In addition, the shape and size of the system should be selected so as to minimize or prevent the drug depot from moving after implantation or injection. Flexibility may be a consideration so as to facilitate placement of the drug depot. In various embodiments, the drug depot can be different sizes, for example, the drug depot may be a length of from about 0.5 mm to 5 mm and have a diameter of from about 0.01 to about 2 mm. The drug depot has a layer thickness of from about 0.005 to 1.0 mm, such as, for example, from 0.05 to 0.75 mm.

The drug depot comprises a ribbon-like fiber, which may be placed at the incision site before the site is closed. The ribbon-like fibers may for example be made of thermosplastic materials. Additionally, specific materials that may be advantageous for use as ribbon-like fibers include the compounds identified above as sustained release biopolymers. The ribbon-like fiber may be formed by mixing the clonidine with the polymer.

Radiographic markers can be included on the drug depot to permit the user to position the depot accurately into the target site of the patient. These radiographic markers will also permit the user to track movement and degradation of the depot at the site over time. In this embodiment, the user may accurately position the depot in the site using any of the numerous diagnostic imaging procedures. Such diagnostic imaging procedures include, for example, X-ray imaging or fluoroscopy. Examples of such radiographic markers include barium, calcium phosphate, and/or metal beads or particles. In various embodiments, the radiographic marker could be a spherical shape or a ring around the depot.

The phrase "sustained release" or "sustain release" (also referred to as extended release or controlled release) is used herein to refer to one or more therapeutic agent(s) that is introduced into the body of a human or other mammal and continuously releases a stream of one or more therapeutic agents over a predetermined time period and at a therapeutic level sufficient to achieve a desired therapeutic effect throughout the predetermined time period. Reference to a continuous release stream is intended to encompass release that occurs as the result of biodegradation *in vivo* of the drug depot, or a matrix or component thereof, or as the result of metabolic transformation or dissolution of the therapeutic agent(s) or conjugates of therapeutic agent(s).

The phrase "immediate release" is used herein to refer to one or more therapeutic agent(s) that is introduced into the body and that is allowed to dissolve in or become absorbed at the location to which it is administered, with no intention of delaying or prolonging the dissolution or absorption of the drug.

### Cannula or Needle

It will be appreciated by those with skill in the art that the depot can be administered to the target site using a cannula or needle that can be a part of a drug delivery device e.g., a syringe, a gun drug delivery device, or any medical device suitable for the application of a drug to a targeted organ or anatomic region. The cannula or needle of the drug depot device is designed to cause minimal physical and psychological trauma to the patient.

Cannulas or needles include tubes that may be made from materials, such as for example, polyurethane, polyurea, polyether(amide), PEBA, thermoplastic elastomeric olefin, copolyester, and styrenic thermoplastic elastomer, steel, aluminum, stainless steel, titanium, metal alloys with high non-ferrous metal content and a low relative proportion of iron, carbon fiber, glass fiber, plastics, ceramics or combinations thereof. The cannula or needle may optionally include one or more tapered regions. In various embodiments, the cannula or needle may be beveled. The cannula or needle may also have a tip style vital for accurate treatment of the patient depending on the site for implantation. Examples of tip styles include, for example, Trephine, Cournand, Veress, Huber, Seldinger, Chiba, Francine, Bias, Crawford, deflected tips, Hustead, Lancet, or Tuohey. In various embodiments, the cannula or needle may also be non-coring and have a sheath covering it to avoid unwanted needle sticks.

The dimensions of the hollow cannula or needle, among other things, will depend on the site for implantation. For example, the width of the epidural space is only about 3-5 mm for the thoracic region and about 5-7 mm for the lumbar region. Thus, the needle or cannula, in various embodiments, can be designed for these specific areas. In various embodiments, the cannula or needle may be inserted using a transforaminal approach in the spinal foramen space, for example, along an inflammed nerve root and the drug depot implanted at this site for treating the condition. Typically, the transforaminal approach involves approaching the intervertebral space through the intervertebral foramina.

Some examples of lengths of the cannula or needle may include, but are not limited to, from about 50 to 150 mm in length, for example, about 65 mm for epidural pediatric use, about 85 mm for a standard adult and about 110 mm for an obese adult patient. The thickness of the cannula or needle will also depend on the site of implantation. In various embodiments, the thickness includes, but is not limited to, from about 0.05 to about 1.655. The gauge of the cannula or needle may be the widest or smallest diameter or a diameter in between for insertion into a human or animal body. The widest diameter is typically about 14 gauge, while the smallest diameter is about 22 gauge. In various embodiments the gauge of the needle or cannula is about 18 to about 22 gauge.

In various embodiments, like the drug depot, the cannula or needle includes dose radiographic markers that indicate location at or near the site beneath the skin, so that the user may accurately position the depot at or near the site using any of the numerous diagnostic imaging procedures. Such diagnostic imaging procedures include, for example, X-ray imaging or fluoroscopy. Examples of such radiographic markers include barium, calcium phosphate, and/or metal beads or particles.

In various embodiments, the needle or cannula may include a transparent or translucent portion that can be visualizable by ultrasound, fluoroscopy, x-ray, or other imaging techniques. In such embodiments, the transparent or translucent portion may include a radiopaque material or ultrasound responsive topography that increases the contrast of the needle or cannula relative to the absence of the material or topography.

The drug depot, and/or medical device to administer the drug may be sterilizable. In various embodiments, one or more components of the drug depot, and/or medical device to administer the drug are sterilized by radiation in a terminal sterilization step in the final packaging. Terminal sterilization of a product provides greater assurance of sterility than from processes such as an aseptic process, which require individual product components to be sterilized separately and the final package assembled in a sterile environment.

Typically, in various embodiments, gamma radiation is used in the terminal sterilization step, which involves utilizing ionizing energy from gamma rays that penetrates deeply in the device. Gamma rays are highly effective in killing microorganisms, they leave no residues nor have sufficient energy to impart radioactivity to the device. Gamma rays can be employed when the device is in the package and gamma sterilization does not require high pressures or vacuum conditions, thus, package seals and other components are not stressed. In addition, gamma radiation eliminates the need for permeable packaging materials.

In various embodiments, electron beam (e-beam) radiation may be used to sterilize one or more components of the device. E-beam radiation comprises a form of ionizing energy, which is generally characterized by low penetration and high-dose rates. E-beam irradiation is similar to gamma processing in that it alters various chemical and molecular bonds on contact, including the reproductive cells of microorganisms. Beams produced for e-beam sterilization are concentrated, highly-charged streams of electrons generated by the acceleration and conversion of electricity. E-beam sterilization may be used, for example, when the drug depot is included in a gel.

Other methods may also be used to sterilize the depot and/or one or more components of the device, including, but not limited to, gas sterilization, such as, for example, with ethylene oxide or steam sterilization.

In various embodiments, a kit is provided that may include additional parts along with the drug depot and/or medical device combined together to be used to implant the drug depot (*e.g*., ribbon-like fibers). The kit may include the drug depot device in a first compartment. The second compartment may include a canister holding the drug depot and any other instruments needed for the localized drug delivery. A third compartment may include gloves, drapes, wound dressings and other procedural supplies for maintaining sterility of the implanting process, as well as an instruction booklet. A fourth compartment may include additional cannulas and/or needles. Each tool may be separately packaged in a plastic pouch that is radiation sterilized. A cover of the kit may include illustrations of the implanting procedure and a clear plastic cover may be placed over the compartments to maintain sterility.

### Drug Delivery

In various embodiments, for the invention provides for the delivery of a therapeutic agent into a surgery site of a patient, the method comprising inserting a cannula at or near a target tissue site and implanting the drug depot at the target site beneath the skin of the patient and brushing, dripping, injecting, or painting the gel in the target site to hold or have the drug depot adhere to the target site. In this way unwanted migration of the drug depot away from the target site is reduced or eliminated.

In various embodiments, to administer the gel having the drug depot dispersed therein to the desired site, first the cannula or needle can be inserted through the skin and soft tissue down to the target tissue site and the gel administered (*e.g*., brushed, dripped, injected, or painted, *etc.)* at or near the target site. In those embodiments where the drug depot is separate from the gel, first the cannula or needle can be inserted through the skin and soft tissue down to the site of injection and one or more base layer(s) of gel can be administered to the target site. Following administration of the one or more base layer(s), the drug depot can be implanted on or in the base layer(s) so that the gel can hold the depot in place or reduce migration. If required a subsequent layer or layers of gel can be applied on the drug depot to surround the depot and further hold it in place. Alternatively, the drug depot may be implanted first and then the gel placed (*e.g*., brushed, dripped, injected, or painted, *etc.)* around the drug depot to hold it in place. By using the gel, accurate and precise implantation of a drug depot can be accomplished with minimal physical and psychological trauma to the patient. The gel also avoids the need to suture the drug depot to the target site reducing physical and psychological trauma to the patient.

In various embodiments, when the target site comprises a spinal region, a portion of fluid (*e.g.,* spinal fluid, *etc.*) can be withdrawn from the target site through the cannula or needle first and then the depot administered (*e.g*., placed, dripped, injected, or implanted, *etc.).* The target site will re-hydrate *(e.g.,* replenishment of fluid) and this aqueous environment will cause the drug to be released from the depot.

Treating or treatment of a disease or condition refers to executing a protocol, which may include administering one or more drugs to a patient (human, other normal or otherwise), in an effort to alleviate signs or symptoms of the disease. Alleviation can occur prior to signs or symptoms of the disease or condition appearing, as well as after their appearance. Thus, "treating" or "treatment" includes "preventing" or "prevention" of disease or undesirable condition. In addition, "treating" or "treatment" does not require complete alleviation of signs or symptoms, does not require a cure, and specifically includes protocols that have only a marginal effect on the patient. By way of example, the administration of the effective dosages of clonidine may be used to prevent, treat or relieve the symptoms of muscle spasticity incidental to surgery through the relaxing of muscle fibers.

"Localized" delivery includes, delivery where one or more drugs are deposited within a tissue, for example, a nerve root of the nervous system or a region of the brain, or in close proximity (within about 10 cm, or preferably within about 5 cm, for example) thereto. "Targeted delivery system" provides delivery of one or more drugs depots, gels or depot dispersed in the gel having a quantity of therapeutic agent that can be deposited at or near the target site as needed for treatment of pain, inflammation or other disease or condition.

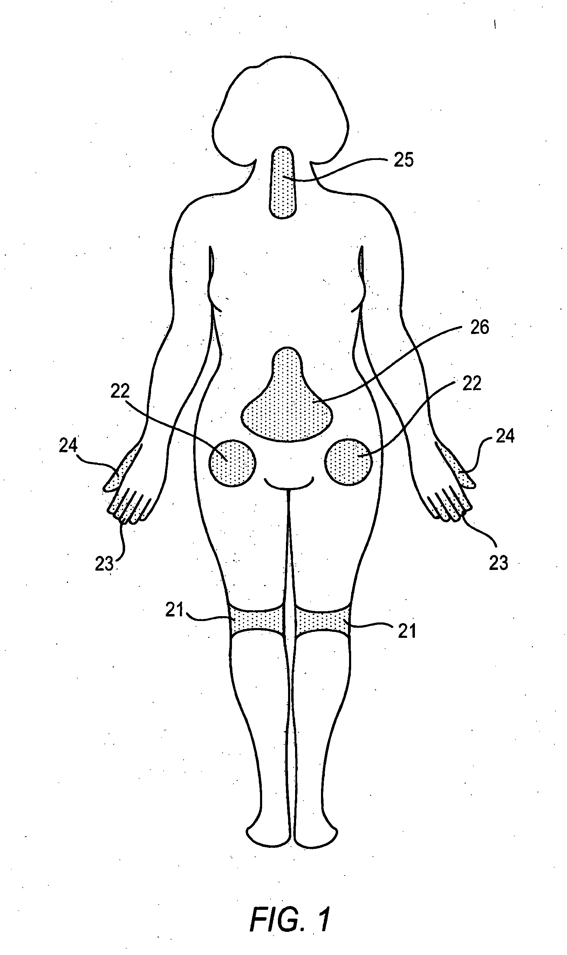

Figure 1 illustrates a number of common locations within a patient that may be sites at which surgery took place. It will be recognized that the locations illustrated in Figure 1 are merely exemplary of the many different locations within a patient that may be at which surgery took place. For example, surgery may be required at a patient's knees 21, hips 22, fingers 23, thumbs 24, neck 25, and spine 26. Thus, during or following these surgeries, the patient may be subject to muscle spasticity.

The term "pain management medication" includes one or more therapeutic agents that are administered to prevent, alleviate or remove pain entirely. These include anti-inflammatory agents, muscle relaxants, analgesics, anesthetics, narcotics, and so forth, and combinations thereof.

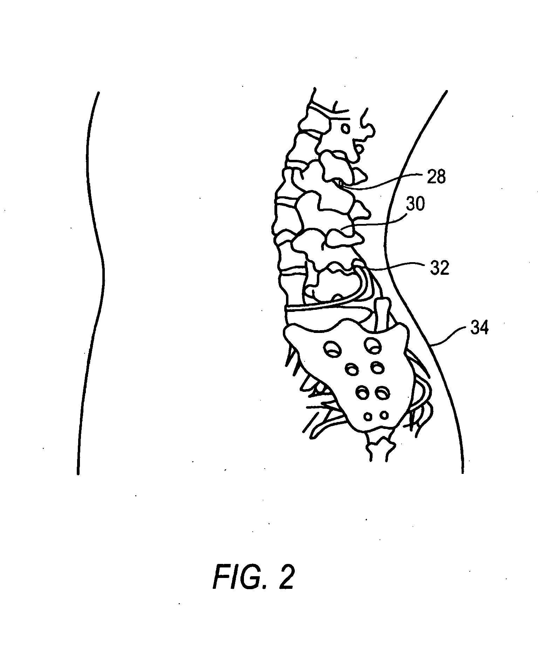

One exemplary embodiment where the depot is suitable for use in pain management (*e.g.,* neuropathic pain management), muscle spasticity treatment, prevention of shivering associated with surgery and/or to treat other conditions (*e.g.,* sciatica) is illustrated in Figure 2. Schematically shown in Figure 2 is a dorsal view of the spine and sites where the drug depot may be inserted using a cannula or needle beneath the skin 34 to a spinal site 32 (*e.g.,* spinal disc space, spinal canal, soft tissue surrounding the spine, nerve root, *etc.)* and one or more drug depots 28 and 32 are delivered to various sites along the spine. In this way, when several drug depots are to be implanted, they are implanted in a manner that optimizes location, accurate spacing, and drug distribution.

Although the spinal site is shown, as described above, the drug depot can be delivered to any site beneath the skin, including at least one muscle, ligament, tendon, cartilage, spinal disc, spinal foraminal space, near the spinal nerve root, or spinal canal.

In some embodiments, it is preferable to co-administer clonidine with an antagonist to counteract undesirable effects, for example the blood pressure decrease that can be caused by clonidine. Exemplary antagonists include phentolamine, yohimbine, tolazoline and piperoxane. Additionally, compounds such as 5-fluorodeoxyuridine (FUDR) and 3,4 dehydroprolene may also be included. These compounds may prevent or reduce glial and fibroblastic scar formation associated with some types of surgeries.

The clonidine-based formulation of the present invention may be used as medicaments in the form of pharmaceutical preparations. The preparations may be formed in an administration with a suitable pharmaceutical carrier that may be solid or liquid as organic or inorganic, and placed in the appropriate form for parenteral or other administration as desired. As persons of ordinary skill are aware, known carriers include water, gelatine, lactose, starches, stearic acid, magnesium stearate, sicaryl alcohol, talc, vegetable oils, benzyl alcohols, gums, waxes, propylene glycol, polyalkylene glycols and other known carriers for medicaments.

The term "mammal" refers to organisms from the taxonomy class "mammalian," including but not limited to humans, other primates such as chimpanzees, apes orangutans and monkeys, rats, mice, cats, dogs, cows, horses, *etc.*

The phrase "release rate profile" refers to the percentage of active ingredient that is released over fixed units of time, *e.g.,* mcg/hr, mcg/day, 10% per day for ten days, *etc.* As persons of ordinary skill know a release rate profile may be but need not be linear. By way of example, the drug depot may be a ribbon-like fiber that releases the clonidine over a period of time.

In some embodiments, the therapeutically effective dosage amount and the release rate profile are sufficient to relax said muscle fibers for a period of 5-12 days; in other embodiments the release rate profile is sufficient to relax the muscle fibers for a period of 7 - 10 days.

The drug depot may also include other active ingredients, including an anti-inflammatory agent, an analgesic agent, a skeletal muscle relaxant, an osteoinductive anabolic growth factor, an anti-catabolic growth factor or a combination thereof. Alternatively, separate drug depots may be co-administered with different active ingredients.

For example, the drug depot may comprise active ingredients in addition to the clonidine. Active ingredients, in various embodiments, block the transcription or translation of TNF-α or other proteins in the inflammation cascade. Suitable active ingredients include integrin antagonists, alpha-4 beta-7 integrin antagonists, cell adhesion inhibitors, interferon gamma antagonists, CTLA4-Ig agonists/antagonists (BMS-188667), CD40 ligand antagonists, Humanized anti-IL-6 mAb (MRA, Tocilizumab, Chugai), HMGB-1 mAb (Critical Therapeutics Inc.), anti-IL2R antibodies (daclizumab, basilicimab), ABX (anti IL-8 antibodies), recombinant human IL-10, or HuMax IL-15 (anti-IL 15 antibodies).

Other suitable active ingredients include IL-1 inhibitors, such Kineret® (anakinra) which is a recombinant, non-glycosylated form of the human inerleukin-1 receptor antagonist (IL-1Ra), or AMG 108, which is a monoclonal antibody that blocks the action of IL-1. Active ingredients also include excitatory amino acids such as glutamate and aspartate, antagonists or inhibitors of glutamate binding to NMDA receptors, AMPA receptors, and/or kainate receptors. Interleukin-1 receptor antagonists, thalidomide (a TNF-α release inhibitor), thalidomide analogues (which reduce TNF-α production by macrophages), bone morphogenetic protein (BMP) type 2 and BMP-4 (inhibitors of caspase 8, a TNF-α activator), quinapril (an inhibitor of angiotensin II, which upregulates TNF-α), interferons such as IL-11 (which modulate TNF-α receptor expression), and aurin-tricarboxylic acid (which inhibits TNF-α), for example, may also be useful as active ingredients for reducing inflammation. It is contemplated that where desirable a pegylated form of the above may be used. Examples of other active ingredients include NF kappa B inhibitors such as glucocorticoids, antioxidants, such as dilhiocarbamate, and other compounds, such as, for example, sulfasalazine.

Anti-inflammatory agents include salicylates, diflunisal, sulfasalazine, indomethacin, ibuprofen, naproxen, tolmetin, ketorolac, diclofenac, ketoprofen, fenamates (mefenamic acid, meclofenamic acid), enolic acids (piroxicam, meloxicam), nabumetone, celecoxib, etodolac, nimesulide, apazone, gold, sulindac or tepoxalin; antioxidants, such as dithiocarbamate, and other compounds such as sulfasalazine [2-hydroxy-5-[-4-[C2-pyridinylamino)sulfonyl]azo]benzoic acid], steroids, such as fluocinolone, cortisol, cortisone, hydrocortisone, fludrocortisone, prednisone, prednisolone, methylprednisolone, triamcinolone, betamethasone, dexamethasone, beclomethasone, fluticasone or a combination thereof.

Suitable anabolic growth or anti-catabolic growth factors include a bone morphogenetic protein, a growth differentiation factor, a LIM mineralization protein, CDMP or progenitor cells or a combination thereof.

Suitable analgesic agents include acetaminophen, lidocaine, bupivicaine, opioid analgesics such as buprenorphine, butorphanol, dextromoramide, dezocine, dextropropoxyphene, diamorphine, fentanyl, alfentanil, sufentanil, hydrocodone, hydromorphone, ketobemidone, levomethadyl, mepiridine, methadone, morphine, nalbuphine, opium, oxycodone, papaveretum, pentazocine, pethidine, phenoperidine, piritramide, dextropropoxyphene, remifentanil, tilidine, tramadol, codeine, dihydrocodeine, meptazinol, dezocine, eptazocine, flupirtine or a combination thereof.

Analgesics also include agents with analgesic properties, such as for example, amitriptyline, carbamazepine, gabapentin, pregabalin, or a combination thereof. The depot may contain a muscle relaxant. Exemplary muscle relaxants include by way of example alcuronium chloride, atracurium bescylate, baclofen, carbolonium, carisoprodol, chlorphenesin carbamate, chlorzoxazone, cyclobenzaprine, dantrolene, decamethonium bromide, fazadinium, gallamine triethiodide, hexafluorenium, meladrazine, mephensin, metaxalone, methocarbamol, metocurine iodide, pancuronium, pridinol mesylate, styramate, suxamethonium, suxethonium, thiocolchicoside, tizanidine, tolperisone, tubocuarine, vecuronium, or combinations thereof.

In some embodiments the clonidine or a portion of the clonidine is administered as a bolus dose at the target tissue to provide an immediate release of the clonidine.

In some embodiments the invention is useful for the treatment of post-operative spasticity and comprises an effective amount of clonidine that is capable of being administered to a post-operative surgery site.

In some embodiments there is a sustain release composition comprising an effective amount of clonidine in an implantable drug depot, wherein said clonidine is present in an amount to relieve spasticity for a period of 5-12 days and said implantable drug depot is present in an amount that facilitates sustained release of clonidine over said period. In some of the embodiments, the period is 7-10 days.

In some embodiments there is an immediate release composition comprising an effective amount of clonidine in a drug depot, wherein said clonidine is present in an amount to relieve spasticity and said drug depot is present in an amount that facilitates immediate release of clonidine.

In some embodiments the compositions are for use in treating a mammal suffering from muscle spasticity associated with surgery, and comprises administering a therapeutically effective amount of clonidine at a target site beneath the skin to relax muscle at or near the target site, wherein said therapeutically effective amount prevents the onset of spasticity. The terms "prevents" is used to refer to a prophylactic administration that is given prior to the onset of the condition. Preferably, the amount is sufficient to and in a form that would mitigate any spasticity that might occur us to 12 days after surgery, e.g., 5-12 days or 7-10 days following surgery.
In some embodiment, there is an implantable drug depot, wherein the drug depot comprises: (i) one or more immediate release layers that release a bolus dose of clonidine or a pharmaceutically acceptable salt thereof at a site beneath the skin; and (ii) one or more sustain release layers that release an effective amount of clonidine or a pharmaceutically acceptable salt thereof over a period of 5 to 12 days.

In some embodiments, the clonidine is first compounded with a polymer to make a first component of the drug depot. In this first compounded product, the clonidine may for example, comprise 0.5% to 20 % of the formulation by weight, 1% to 15% of the formulation by weight or 2.5% to 10% of the formulation by weight. The remainder of the formulation may be exclusively polymer or comprises excipients, surfactants or other inactive ingredients.

In some embodiments, the dosage is from approximately 0.0005 to approximately 100 µg/kg/day. Additional dosages of clonidine can include from approximately 0.0005 to approximately 95 µg/kg/day; approximately 0.0005 to approximately 90 µg/kg/day; approximately 0.0005 to approximately 85 µg/kg/day; approximately 0.0005 to approximately 80 µg/kg/day; approximately 0.0005 to approximately 75 µg/kg/day; approximately 0.001 to approximately 70 µg/kg/day; approximately 0.001 to approximately 65 µg/kg/day; approximately 0.001 to approximately 60 µg/kg/day; approximately 0.001 to approximately 55 µg/kg/day; approximately 0.001 to approximately 50 µg/kg/day; approximately 0.001 to approximately 45 µg/kg/day; approximately 0.001 to approximately 40 µg/kg/day; approximately 0.001 to approximately 35 µg/kg/day; approximately 0.0025 to approximately 30 µg/kg/day; approximately 0.0025 to approximately 25 µg/kg/day; approximately 0.0025 to approximately 20 µg/kg/day; and approximately 0.0025 to approximately 15 µg/kg/day. In another embodiment, the dosage of clonidine is from approximately 0.005 to approximately 15 µg/kg/day. In another embodiment, the dosage of clonidine is from approximately 0.005 to approximately 10 µg/kg/day. In another embodiment, the dosage of clonidine is from approximately 0.005 to approximately 5 µg/kg/day. In another embodiment, the dosage of clonidine is from approximately 0.005 to approximately 20 µg/kg/day. In some embodiments, the dose is 200 to 600 mg per day and is applied to a human.

In some embodiments, it is desirable to use a sufficient amount of clonidine loaded in the drug depot to achieve a plasma or blood level of at least 1 microgram per kilogram of bodyweight, or at least about 2 - 8 micrograms per kilogram of body weight, or at least about 3-6 micrograms per kilogram of bodyweight. Further, in some embodiments, it is desirable to use a sufficient amount of clonidine in the drug depot in a single sustained release formulation to sustain these plasma or blood levels for up to 12 days after surgery, e.g., 5-12 days or 7-10 days.

In some embodiments, the clonidine is administered parenterally, e.g., by injection. In some embodiments, the injection is intrathecal, which refers to an injection into the spinal canal (intrathecal space surrounding the spinal cord). An injection may also be into a muscle or other tissue. In other embodiments, the clonidine is adminstered by placement into an open patient cavity during surgery itself.

## Claims

1. Clonidine or a pharmaceutically acceptable salt thereof for use in treating a mammal suffering from muscle spasticity associated with surgery, said use comprising administering a therapeutically effective amount of the clonidine or the pharmaceutically acceptable salt thereof and a biodegradable polymer selected from D,L-lactide or L-lactide-caprolactone at a target tissue site beneath the skin to relax muscle at or near the target tissue site wherein clonidine is for administration locally to the target tissue site as a drug depot with a layer thickness of from 0.005mm to 1.0mm in the form of a ribbon-like fiber that releases the clonidine over a period of time.

2. Clonidine or a pharmaceutically acceptable salt thereof for use according to claim 1, wherein the drug depot further comprises an anti-inflammatory agent, an analgesic agent, a skeletal muscle relaxant, an osteoinductive anabolic growth factor, an anti-catabolic growth factor or a combination thereof.

3. Clonidine or a pharmaceutically acceptable salt thereof for use according to claim 1, comprising an integrin antagonist.

4. Clonidine or a pharmaceutically acceptable salt thereof for use according to claim 1 , wherein said therapeutically effective amount and said release are sufficient to relax said muscle for a period of 7 - 10 days.

5. Clonidine or a pharmaceutically acceptable salt thereof for use according to claim 1, wherein said administering comprises introducing a bolus dose of clonidine at the target tissue site.

6. Clonidine or a pharmaceutically acceptable salt thereof for use according to claim 1 in the form of a sustained release composition comprising an effective amount of clonidine or a pharmaceutically acceptable salt thereof in an implantable drug depot, wherein said clonidine or a pharmaceutically acceptable salt thereof is present in an amount to relieve post-operative spasticity for a period of 5-12 days and wherein said implantable drug depot facilitates sustain release of clonidine over said period.

7. Clonidine or a pharmaceutically acceptable salt thereof for use according to claim 1 which is an immediate release composition comprising an effective amount of clonidine or a pharmaceutically acceptable salt thereof in a drug depot, wherein said clonidine or a pharmaceutically acceptable salt thereof is present in an amount to relieve post-operative muscle spasticity and said drug depot is present in an amount that facilitates immediate release of clonidine.

8. Clonidine or a pharmaceutically acceptable salt thereof for use according to claim 1 in treating a mammal suffering from muscle spasticity associated with surgery, said use comprising administering a therapeutically effective of the clonidine or a pharmaceutically acceptable salt thereof at a target tissue site beneath the skin to relax muscle at or near the target tissue site, wherein said therapeutically effective amount prevents the onset of spasticity.

9. Clonidine or a pharmaceutically acceptable salt thereof for use according to claim 1, wherein said administering comprises introducing a bolus dose of clonidine at the target tissue to provide an immediate release of the clonidine.

10. Clonidine or a pharmaceutically acceptable salt thereof for use according to claim 1, wherein the drug depot comprises i) one or more immediate release layers that release a bolus dose of clonidine or a pharmaceutically acceptable salt thereof at a site beneath the skin and ii) one or more sustain release layers that release an effective amount of clonidine or a pharmaceutically acceptable salt over a period of 5 to 12 days.

11. Clonidine or a pharmaceutically acceptable salt thereof for use according to claim 1, for use in the treatment of post-operative muscle spasticity.

## Patentansprüche

1. Clonidin oder ein pharmazeutisch akzeptables Salz davon zur Verwendung bei der Behandlung eines Säugetiers, das an mit einem chirurgischen Eingriff assoziierter Muskelspastizität leidet, wobei die genannte Verwendung das Verabreichen einer therapeutisch wirksamen Menge des Clonidins oder des pharmazeutisch akzeptablen Salzes davon und eines biologisch abbaubaren Polymers, ausgewählt aus D,L-Lactid oder L-Lactid-Caprolacton, an eine Zielgewebestelle unter der Haut beinhaltet, um Muskeln an oder nahe der Zielgewebestelle zu entspannen, wobei Clonidin zur lokalen Verabreichung an die Zielgewebestelle als Arzneimitteldepot mit einer Schichtdicke von 0,005 mm bis 1,0 mm in Form einer bandähnlichen Faser dient, die das Clonidin über eine Zeitperiode freisetzt.

2. Clonidin oder ein pharmazeutisch akzeptables Salz davon zur Verwendung nach Anspruch 1, wobei das Arzneimitteldepot ferner ein entzündungshemmendes Mittel, ein analgetisches Mittel, ein Skelettmuskelentspannungsmittel, einen osteoinduktiven anabolischen Wachstumsfaktor, einen anti-katabolischen Wachstumsfaktor oder eine Kombination davon beinhaltet.

3. Clonidin oder ein pharmazeutisch akzeptables Salz davon zur Verwendung nach Anspruch 1, das einen Integrinantagonisten umfasst.

4. Clonidin oder ein pharmazeutisch akzeptables Salz davon zur Verwendung nach Anspruch 1, wobei die genannte therapeutisch wirksame Menge und die genannte Freisetzung zum Entspannen des genannten Muskels für eine Periode von 7 - 10 Tagen ausreichen.

5. Clonidin oder ein pharmazeutisch akzeptables Salz davon zur Verwendung nach Anspruch 1, wobei das genannte Verabreichen das Einleiten einer Clonidin-Bolusdosis an der Zielgewebestelle beinhaltet.

6. Clonidin oder ein pharmazeutisch akzeptables Salz davon zur Verwendung nach Anspruch 1 in Form einer Zusammensetzung mit anhaltender Freisetzung, die eine wirksame Menge an Clonidin oder einem pharmazeutisch akzeptablen Salz davon in einem implantierbaren Arzneimitteldepot umfasst, wobei das genannte Clonidin oder ein pharmazeutisch akzeptables Salz davon in einer Menge vorhanden ist, die eine postoperative Spastizität für eine Periode von 5-12 Tagen lindert, und wobei das genannte implantierbare Arzneimitteldepot die anhaltende Freisetzung von Clonidin über die genannte Periode unterstützt.

7. Clonidin oder ein pharmazeutisch akzeptables Salz davon zur Verwendung nach Anspruch 1, die eine Zusammensetzung mit sofortiger Freisetzung ist, die eine wirksame Menge an Clonidin oder einem pharmazeutisch akzeptablen Salz davon in einem Arzneimitteldepot beinhaltet, wobei das genannte Clonidin oder ein pharmazeutisch akzeptables Salz davon in einer Menge vorhanden ist, die postoperative Muskelspastizität lindert, und das genannte Arzneimitteldepot in einer Menge vorhanden ist, die eine sofortige Freisetzung von Clonidin unterstützt.

8. Clonidin oder ein pharmazeutisch akzeptables Salz davon zur Verwendung nach Anspruch 1 bei der Behandlung eines Säugetiers, das an mit einem chirurgischen Eingriff assoziierter Muskelspastizität leidet, wobei die genannte Verwendung das Verabreichen einer therapeutisch wirksamen Menge des Clonidins oder eines pharmazeutisch akzeptablen Salzes davon an eine Zielgewebestelle unter der Haut beinhaltet, um Muskeln an oder nahe der Zielgewebestelle zu entspannen, wobei die genannte therapeutisch wirksame Menge das Einsetzen von Spastizität verhütet.

9. Clonidin oder ein pharmazeutisch akzeptables Salz davon zur Verwendung nach Anspruch 1, wobei das genannte Verabreichen das Einleiten einer Clonidin-Bolusdosis an dem Zielgewebe beinhaltet, um eine sofortige Freisetzung des Clonidins zu bewirken.

10. Clonidin oder pharmazeutisch akzeptables Salz davon zur Verwendung nach Anspruch 1, wobei das Arzneimitteldepot i) eine oder mehrere Schichten mit sofortiger Freisetzung, die eine Bolusdosis von Clonidin oder einem pharmazeutisch akzeptablen Salz davon an einer Stelle unter der Haut freisetzen, und ii) eine oder mehrere Schichten mit anhaltender Freisetzung umfasst, die eine wirksame Menge an Clonidin oder einem pharmazeutisch akteptablen Salz über eine Periode von 5 bis 12 Tagen freisetzen.

11. Clonidin oder pharmazeutisch akzeptables Salz davon zur Verwendung nach Anspruch 1, zur Verwendung bei der Behandlung von postoperativer Muskelspastizität.

## Revendications

1. Clonidine ou sel pharmaceutiquement acceptable de celle-ci, pour une utilisation dans le traitement d'un mammifère souffrant d'une spasticité musculaire associée à une chirurgie, ladite utilisation comprenant l'administration d'une quantité thérapeutiquement efficace de la clonidine ou du sel pharmaceutiquement acceptable de celle-ci et d'un polymère biodégradable choisi parmi la D,L-lactide- ou L-lactide-caprolactone, au niveau d'un site tissulaire cible sous la peau afin de relâcher les muscles au niveau du site tissulaire cible ou à proximité de celui-ci, où la clonidine est destinée à une administration locale au niveau du site tissulaire cible sous forme de dépôt médicamenteux ayant une épaisseur de couche allant de 0,005 mm à 1,0 mm, sous la forme d'une fibre semblable à un ruban qui libère la clonidine sur une période de temps.

2. Clonidine ou sel pharmaceutiquement acceptable de celle-ci, pour une utilisation selon la revendication 1, dans laquelle le dépôt médicamenteux comprend en outre un agent anti-inflammatoire, un agent analgésique, un relaxant des muscles squelettiques, un facteur de croissance anabolique ostéo-inducteur, un facteur de croissance anti-catabolique ou une combinaison de ceux-ci.

3. Clonidine ou sel pharmaceutiquement acceptable de celle-ci, pour une utilisation selon la revendication 1, comprenant un antagoniste de l'intégrine.

4. Clonidine ou sel pharmaceutiquement acceptable de celle-ci, pour une utilisation selon la revendication 1, dans laquelle ladite quantité thérapeutiquement efficace et ladite libération sont suffisantes pour relâcher ledit muscle pour une période de 7-10 jours.

5. Clonidine ou sel pharmaceutiquement acceptable de celle-ci, pour une utilisation selon la revendication 1, dans laquelle ladite administration comprend l'introduction d'une dose bolus de clonidine au niveau du site tissulaire cible.

6. Clonidine ou sel pharmaceutiquement acceptable de celle-ci, pour une utilisation selon la revendication 1, sous la forme d'une composition à libération prolongée comprenant une quantité efficace de clonidine ou d'un sel pharmaceutiquement acceptable de celle-ci dans un dépôt médicamenteux implantable, où ladite clonidine ou un sel pharmaceutiquement acceptable de celle-ci est présent(e) selon une quantité destinée à soulager une spasticité postopératoire pendant une période de 5-12 jours et où ledit dépôt médicamenteux implantable facilite une libération prolongée de clonidine au cours de ladite période.

7. Clonidine ou sel pharmaceutiquement acceptable de celle-ci, pour une utilisation selon la revendication 1, qui est une composition à libération immédiate comprenant une quantité efficace de clonidine ou d'un sel pharmaceutiquement acceptable de celle-ci dans un dépôt médicamenteux, où ladite clonidine ou un sel pharmaceutiquement acceptable de celle-ci est présent(e) selon une quantité destinée à soulager une spasticité musculaire postopératoire et ledit dépôt médicamenteux est présent selon une quantité qui facilite une libération immédiate de clonidine.

8. Clonidine ou sel pharmaceutiquement acceptable de celle-ci, pour une utilisation selon la revendication 1, pour le traitement d'un mammifère souffrant d'une spasticité musculaire associée à une chirurgie, ladite utilisation comprenant l'administration d'une quantité thérapeutiquement efficace de la clonidine ou d'un sel pharmaceutiquement acceptable de celle-ci au niveau d'un site tissulaire cible sous la peau afin de relâcher les muscles au niveau du site tissulaire cible ou à proximité de celui-ci, où ladite quantité thérapeutiquement efficace empêche le déclenchement d'une spasticité.

9. Clonidine ou sel pharmaceutiquement acceptable de celle-ci, pour une utilisation selon la revendication 1, dans laquelle ladite administration comprend l'introduction d'une dose bolus de clonidine au niveau du tissu cible afin de fournir une libération immédiate de la clonidine.

10. Clonidine ou sel pharmaceutiquement acceptable de celle-ci, pour une utilisation selon la revendication 1, dans laquelle le dépôt médicamenteux comprend i) une ou plusieurs couches de libération immédiate qui libèrent une dose bolus de clonidine ou d'un sel pharmaceutiquement acceptable de celle-ci au niveau d'un site sous la peau et ii) une ou plusieurs couches de libération prolongée qui libèrent une quantité efficace de clonidine ou d'un sel pharmaceutiquement acceptable sur une période allant de 5 à 12 jours.

11. Clonidine ou sel pharmaceutiquement acceptable de celle-ci, pour une utilisation selon la revendication 1, pour une utilisation dans le traitement d'une spasticité musculaire postopératoire.
